(19) Europäisches Patentamt
European Patent Office
Office européen des brevets

(11) Publication number : **0 157 753**
**B1**

(12) # EUROPEAN PATENT SPECIFICATION

(45) Date of publication of patent specification : **11.11.87**

(21) Application number : **85870041.2**

(22) Date of filing : **20.03.85**

(51) Int. Cl.[4] : **C 07 K 9/00**, A 61 K 37/00, A 61 K 39/00// C07H13/04, C07H15/18

(54) **New somatostatin compounds, process for their synthesis, preparation for veterinary use containing said compound and process for the treatment of animals.**

(30) Priority : **26.03.84 LU 85269**

(43) Date of publication of application : **09.10.85 Bulletin 85/41**

(45) Publication of the grant of the patent : **11.11.87 Bulletin 87/46**

(84) Designated contracting states : **AT BE CH DE FR GB IT LI LU NL SE**

(56) References cited :
**EP-A- 0 089 290**
**LA RECHERCHE, vol. 14, no. 142, March 1983, pages 346-357, Paris, FR; R. ARNON et al.: "Les antigènes et vaccins synthétiques"**

(73) Proprietor : **TECHLAND, Société Anonyme**
**Parc Industriel de Recherches du Sart-Tilman Allée des Noisetiers**
**B-4900 Liège-Angleur (BE)**

(72) Inventor : **Hennen, Georges Paul Guillaume**
**Havelange, 15**
**B-4071 Harze (BE)**
Inventor : **Closset, Jean Lambert René**
**Rue Renardi, 111**
**B-4000 Liege (BE)**
Inventor : **Tran Quang, Mihn dit Sato**
**Rue Lambert Dewonck, 147**
**B-4430 Alleur/Ans (BE)**

(74) Representative : **van Malderen, Michel et al**
**p.a. FREYLINGER & ASSOCIES 85/042 Boulevard de la Sauvenière**
**B-4000 Liège (BE)**

**EP 0 157 753 B1**

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Jouve, 18, rue St-Denis, 75001 Paris, France

## Description

### Background of the invention

1. Field of the Invention

The present invention is related to new somatostatin compounds constituting a synthetic vaccine applicable for the treatment of animals, a synthesis process for the compounds, a preparation for veterinary use containing said new compounds and a process for the treatment of animals.

2. Description of the Prior Art

Some anabolizing hormonal compounds (steroids) have been used in stock rearing in order to promote the weight gain of cattle. The treatment by means of implanted steroids or analogs has the major drawbăck that the toxic residues remain in the animal tissues and the administration of these substances has been forbidden in many countries.

R. Arnon et al : Les antigènes et vaccins synthétiques, La Recherche N° 142, March 1983, Vol. 14, pp. 346-357 Paris, France, describe synthetic vaccines and the use of N-acetylmuramyl-L-alanyl-D-isoglutamine (or MDP for muramyl-dipeptide) and homologues thereof in such synthetic vaccines e. g. to increase the antigenic nature of P2-A-L to which it is bound in a covalent manner. Said article also describes a synthetic antigenic material resulting from MDP and a synthetic peptide having the sequence of the hormone responsible in the males for the regulation of the expression of testosterone. The conjugate has been experienced on male mice.

In the publication EP-A-0 089 290 (ANVAR), the same technique has been used to prepare conjugates of MDP and hormones of various nature. The publication describes essentially veterinary vaccines allowing an increase of meat production by an immunologic castration obtained by a conjugate of MDP with human chorionic gonadotropine (hCG).

The present invention aims accordingly to avoid the cited drawbacks of the administration of anabolizing or of castrating compounds and includes accordingly a new approach to the promotion of growth and/or milk production of domestic animals.

### Summary of the invention

The growth hormone also called somatotropin is the main hormone associated with the post-natal growth. It is known that the secretion of this hormone is regulated by two neuropeptides, on the one hand somatostatin which inhibits the release of the growth hormone and on the other hand somatocrinin which stimulates the synthesis and the secretion of the growth hormone.

The principle on which the present invention rests, consist in a vaccinal preparation valuable for inhibiting or neutralizing the endogenous somatostatin of the treated animal.

The active ingredient of new vaccine of the present invention is a compound resulting from coupling of the N-acetylmuramyl-L-alanyl- D-isoglutamine or a homologue thereof with somatostatin or a homologue in the natural or oxidized state or in the pro-form or its pre-proforms.

The term homologues is thereafter defined.

The process, using said vaccine (GPP : Growth promoting peptide) or any of its derivatives is designed to increase growth and development of domestic animals of various species, among which and more specifically the bovine, for meat production.

The first degree of the procedure is to directly immunize by the GPP or its derivatives the recipient animal for which it is intended to increase growth and development.

The term derivative is thereafter defined.

### Detailed description of the invention

The immunization scheme results in various types of antibodies :

1. a first order are potentially directed against epitopes represented by somatostatin or its homologues. The coupling methods of somatostatin or its homologues have indeed been designed to keep intact the greatest number of epitopes expressed by these peptides.

2. a second order are antibodies directed by epitopes created by the GPP itself or its homologues and specific to these new types of coupled molecules. These antibodies are nevertheless capable of cross-reacting with endogenous material such as or similar to somatostatin or muramyl peptides.

3. a third order are antibodies reacting selectively with epitopes created by the muramyl peptides.

It is presumed, in view of our experimental data, that these various antibodies are all potentially involved in the observed phenomena, i. e. increased development of the immunized animals.

To achieve the effects, the antibodies have to neutralize biological endogenous materials, which are thus less capable or uncapable of directing an effect, the net result being an increase in growth and muscle development.

Conversely, the antibodies could interact with substances present in the animal, either as endogenous material or beared by hosts or parasites of the animals. Through the interaction, effects adverse to growth and development are neutralized through humoral or humoral and cellular immunity. The affinity constant (Ka) of the association reaction between the antibodies and above described antigenic materials have to reach a magnitude ranging from $10^{-7}$ L/M to $10^{-13}$ L/M.

The process of increasing growth and development of animals can also be achieved by passive immunization. In that case, immunoglobulins of the required characteristics such as listed above, could be elicited in recipient animals and purified from their biological fluids. The necessary antibodies can also be obtained through the cellular fusion techniques, hybridoma selection and culture, harvesting culture medium and ascitic fluids and purification of the monoclonal antibodies possessing the required characteristics. An approach by recombinant DNA technology can also be used to produce the required antibodies by genetic engineering.

The vaccine may specifically be the compound resulting from the coupling of an ester, e. g. the $C_1$-$C_{10}$ alkyl ester of N-acetylmuramyl-L-alanyl-D-isoglutaminyl-L-lysine at the carboxylic group of somatostatin or an ester of somatostatin e. g. the $C_1$-$C_{10}$ alkylester thereof at the carboxylic end of MDP in the natural or oxidized states or in the pro-form. The preferred compounds of the invention obtained in this manner have the formula I or II hereafter :

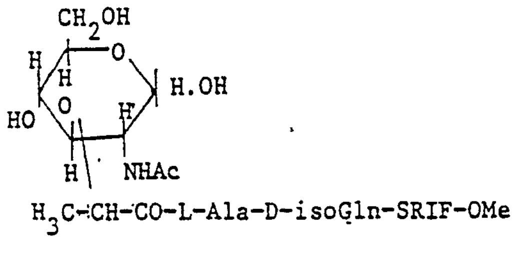

(I)

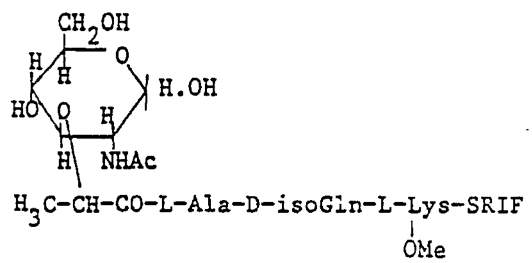

(II)

wherein SRIF represents somatostatin or a homologue thereof. The compounds of the invention may be administrated in appropriate form e. g. in the form of a preparation for veterinary use in a physiological liquid containing an excipient such as polyethylenglycol in the proportion of 150 mg in 100 ml.

The procedures of immunization is based on a single or multiple immunizing injections preferably a parenteral injection containing the GPP or its homologs in an effective quantity which can vary from 10 to 500 μg per dose depending upon the species of the recipient and the type of procedure chosen (active or passive immunization).

Said parenteral injection may be e. g. an intramuscular or subcutaneous injection possibly followed by one or more boosting injections.

However other modes of administration such as oral or a nasal spray administration are also contemplated by the present invention.

Similarly, the antibodies which can be passively administrated to the recipient animal would be administered in doses varying from at least 5 mg/kg body weight in variable doses, e. g. from 1 to 5 doses at an interval of e. g. several days.

It is understood that the growth promoting mechanism could involve anti-idiotype antibodies. These could be responsible partially or totally to the observed effect on these intended to be produced.

Any suitable excipient which would respect or enhance the immunogenic properties of the vaccine or of the vaccine vehiculated by an immunostimulating complex is part of the invention.

The dosage of active ingredient in the compositions of this invention may be varied, however it is necessary that the amount of the active ingredient shall be such that a suitable dosage form is obtained. The selected dosage depends upon the desired therapeutic effect, on the route of administration, and on the duration of the treatment.

Advantageous pharmaceutical compositions are constituted by injectable solutions or suspensions containing an effective dose of at least one product according to the invention. Preferably, these solutions or suspensions are formed in an isotonic sterilized aqueous phase preferably saline or glucosed. The invention relates more particularly to such suspensions or solutions which are suitable for administration by intradermal, intramuscular or sub-cutaneous injection, or again by scarification and notably pharmaceutical composition in the form of liposomes whose constitution is well known.

It relates also to pharmaceutical compositions administrable by other routes, notably by the oral or rectal route, or again in the form of aerosols designed to be applied to the mucous membranes, notably the ocular, nasal, pulmonary or vaginal mucous membranes.

In consequence, it relates to pharmaceutical compositions in which one at least of the compounds according to the invention is associated with pharmaceutically acceptable excipients, solid or liquid,

adapted to the constitution of oral, ocular or nasal forms, or with excipients adapted for the constitution of rectal forms of administration, or again with gelatinous excipients for vaginal administration. It relates also to isotonic liquid compositions containing one at least of the products according to the invention, adapted for administration to the mucous membranes, notably the ocular to nasal mucous membranes.

It relates lastly to compositions formed of pharmaceutically acceptables liquified gases of the « propellant » type, in which the products according to the invention are dissolved or held in suspension, and of which the release causes the dispersions in an aerosol.

Preparations according to this invention for parental administrations include sterile aqueous or non aqueous solutions, suspensions or emulsions. Examples of non aqueous solvents or vehicles are propylene glycol, polyethylene glycol, vegetable oils, such as olive oil and corn oil, gelatin and injectable organic esters such as ethyl oleate. Such dosage forms may also contain adjuvant such as preserving, wetting, emulsifying and dispersing agents. They may be sterilized by, for example, filtration through a bacteria-retaining filter, by incorporating sterilizing agents into the compositions, by irradiating the compositions, or by heating the compositions. They can also be manufactured in the form of sterile water, or some other sterile injectable medium immediatly before use. Compositions for rectal or vaginal administration are preferably suppositories which may contain, in addition to the active substance, excipients such as cocoa butter or a suppository wax.

Composition for nasal or sublingual administration are also prepared with standard excipients well known in the art.

The compounds are useful in a process for the treatment of domestic animals in view of stimulating their growth and/or the production of milk.

The technique has a series of advantages, compared to known techniques, among which one may cite :

The increase of weight gain and the weight production results directly from an increased secretion of the endogenic growth hormone.

Due to the similarity, i. e. the homology of the structure of somatostatin or its homologues for several domestic animals, the vaccine may be used for a great variety of animals, without structural modification.

The technique, compared to other suggested procedures, is easily carried out and requires only a limited number of injections. Furthermore, the treatment by means of the proposed vaccine, produces the same effects regarding weight increase and milk production than the treatments by means of exogenic growth hormone which is expensive and difficult to obtain or to produce and which furthermore causes difficulties for a slow and regular release of the exogenic hormone in the treated animals. It does further not interfere with the HCG system, and causes no immunologic castration.

The starting materials for the preparation of the vaccine can be easily obtained and are relatively cheap. The techniques which are used for the synthesis of the vaccine are well defined, reproductible and in the reach of normally equipped laboratories for the synthesis of peptides.

The use of the vaccine of the invention allows to avoid the difficult studies which are necessary for the use of implants and the necessity of producing the growth hormone in great quantity, in particular by the techniques of genetic engineering.

The proposed technique also avoids the mass production by the genetic way, in particular of the specific growth hormone for each species. It is moreover known that the administration of a growth hormone prepared by means of pituitary bodies collected in slaughter-houses is as not commercially profitable treatment.

The highly immuno reactive characteristic of the vaccine of the invention allows a limited number of injections.

The growth promoting peptide of the invention may thus be regarded as a mean which is easy, efficient, relatively non expensive and safe to reach the wished aim.

## Preparation of products according to the invention

The process for coupling N-acetylmuramyl-L-alanyl-D-isoglutamine or a homologues with somatostatin or a homologues in the natural or oxidized state or in the pro-form or its pre-proforms may be performed by any of the methods well known in the art.

Such methods have for instance been described in Publications EP-A-0 003 833 (Ciba-Geigy) and its counterparts AU-A-1 248 583 and JP-A-58 208 237 which are incorporated herein by reference.

In carrying out the process, it appeared that the ratio of N-acetylmuramyl-L-alanyl-D-isoglutamine or a homologue thereof to somatostatin or a homologue thereof may be varied in several ratios comprised between 1/1 and 3/1 while exercing effects which are qualitively similar.

## Definition of homologues and derivatives

On the basis of the original immunogen molecular structure (GPP, muramyl di or tripeptide) a series of homologues would result from :

a. Homologues to the somatostatin portion,

b. Homologues of muramyl peptide(s),

c. Compounds resulting from the original portion of the molecule or their homologues but created by different coupling, thereafter called derivatives.

These homologues or derivatives could be used to increase growth and meat production with an efficiency which compares at various degrees with that of original GPP(s).

Homologues at the level of the somatostatin portion :

On the model of the oxydized 14 amino acid polypeptide, substitution of any of those 14 residues could be replaced by a D- instead of the initial L-form. Typical analogs are a D-tryptophanyl residue in position 8, D-bromo or fluoro-Trp in the same position.

A radical change in the nature of all amino-acids can be obtained in any position of the chain by a natural or non natural L- or D- aminoacid (taurine or aminobutyric acid for example).

DES-forms are also usable as well as cyclic forms (non reductible cyclic analogs). Pro-forms, such as S.S. 28 amino acids form and SS DES-25 proform under their reduced or oxydized forms are usable.

Other forms with additional $NH_2$-terminal aminoacid resulting from peptide synthesis or DNA recombinant techniques could be used either as a molecule presenting a portion of the natural presequence or as molecules exhibiting any sort of substitution in any position of this proform.

Molecules resulting from the protection of reactive groups on the side chains of the aminoacids are also usable such as :

for the OH-groups :

acetyl, benzoyl, terbutyl, trityl, tetrahydro pyramyl, benzyl, 2,6-dichlorobenzyl, benzyloxycarbonyl, guanyl, nitroso, tosyl.

for E-$NH_2$ groups :

paramethoxybenzyl, paramethylbenzyl, acetamidomethyl, tritil and benzyl, benzyloxycarbonyl, adamentyloxy carbonyl, terbutyloxycarbonyl.

for the COOH groups :

methyl, ethyl, benzyl, dichlorobenzyl or any other esters, up to ten atoms of long aliphatic chain, amide or azide.

Glycosylated somatostatin analog could result from the glycosylation of 5-asparagine, 13-serine, 10- and 12-threonine or any other position provided that this position is occupied by one of these 3 amino acids.

Substitution of sulfur in cysteine residue by selenium atome would yield a somatostatin homologue in its reduced and oxydized form.

Additional derivatisation of the somatostatin portion of any GPP could result from the use of a polymer of various order of somatostatin itself retaining at least one and preferably multiple immunogenic determinants of somatostatin.

Homologues at the level of the muramyl peptide.

A serie of homologues would automatically result from the method used for the synthesis of the muramyl peptide by protecting hydroxyl groups, essentially by benzyl and benzylidene.

Substitution of hydrogen of 1-hydroxy group or 1-hydroxy of the saccharide ring could be performed by alkyl, aryl, alkylaryl, arylalkyl or by amino group. These substitutes would have functional groups such as hydroxy, amine, carboxamide, sulfonamide, ether, thioether. Preferentially 4-carbone would bear hydrogen atom and hydroxyl groups and oxyacyl could be present. 2-hydroxyl group of saccharide ring could be substituted by acetamido or glycolyl amino groups.

Substitution of hydrogen of 6-hydroxy function or 6-hydroxy of the saccharide ring could be protected by carboxamide, sulfonamide, ether, thioether, ester, thioester or replaced by amine. These groups could possibly bear aryl, phenyl, heterocyclic, alkyl, carbonyl, amino, sulfhydryl, peroxyl groups.

A typical substitution of H atom of 6-hydroxy could be achieved by fatty acids to create a 6-0-stearic homologue of muramyl peptides for example. This type of homologue could also be inserted into a liposome to create a particularly active immunogen.

In the peptide moiety of the muramyl peptide, the alanyl residue could be substituted by arginyl, asparagyl, aspartyl, glutamyl, glycyl, histidyl, hydroxyprolyl, isoleucyl, leucyl, lysyl, methionyl, phenylalanyl, prolyl, seryl, threonyl, tryptophanyl, tyrosyl, valyl, aminobutyryl residues.

The carboxyl-function of the D-isoglutaminyl residue could also be esterified or substituted by any

other amino acid having an other carboxylic function. The lysyl residue of the muramyl tripeptide could be substituted by an amino acid having supplementary to their amino and carboxylic functions, an amino, hydroxyl or acid function. Such amino acid could be aspartic acid, glutamic acid, ornithine, tyrosine.

Derivatives resulting from alternative coupling of MDP and homologues with somatostatin or homologues.

The procedures to obtain alternative coupling involve functional groups present on one of the reaction partners such as amine, carboxyl, sulfhydryl, alcohol, reactive ester and the complementary reacting group on the other reaction partner.

The reaction is made in the presence of an appropriate condensation reagent. Before the reaction, protecting groups could be introduced on all functional groups which would not be involved in the reaction.

The coupling reaction could be made with MDP homologues substituted preferentially on positions 1 and 6 of the saccharide portion of the MDP or homologues by groups like phenylisocyanate and diazonium respectively. These reagents would react with the free amine group of somatostatin or homologues.

In the case in which the only reacting group possessed by the reaction partner would be free amine groups, coupling reaction would be realized using glutaraldehyde or benzoquinone.

Photoactivated coupling can be used to conjugate somatostatin homologues devoid of reacting groups.

This can include the substitution on the osidic cycles or on the peptide side chain of the MDP or homologues by nitrene or carbene functions.

These photoactivated reactions can lead to insertion in the aliphatic, saturated or non saturated chains present in the somatostatin or its homologues.

Coupling can also be made when somatostatin or its homologues and MDP and its homologues possess one free SH group and one maleimide function.

An alternate coupling method can result from the reaction between a carboxyl group present in one partner and an amine group in the second partner of the reaction. This will create derivatives possessing N-hydroxysuccinimide ester function. The reacting carboxyl group will either present naturally or placed by synthesis either on the saccharidic or peptidic portion of the MDP or its homologues or somatostatin or its homologues. Also usable is the method utilizing mixed anhydrides (alkylchloro- carbonate in a alkaline medium). This methode creates a bond between a carboxyl group present in one partner with an amine group present in the other partner. This reaction is being carried out preferentially in anhydrous medium.

Coupling through a Schiff base is possible when glycozylated homologues of the peptide portion of MDP or its homologues or of somatostatin or its homologues react with a functional amine group present on the other partner or any of its homologues.

Examples and synthesis methods for somatostatin homologues are described in the following publications, which are given for illustration purpose only, said homologues being well known to those specialized in the art.

US-A-4 317 711 (Shiba et al)
US-A-4 396 607 (Lefrancier et al)
US-A-4 372 884 (Brown et al)
US-A-4 223 020 (Momany)
US-A-4 212 795 (Hughes et al)
EP-A-0 006 068 (Agence Nationale)
EP-A-0 056 560 (Ciba-Geigy)
EP-A-0 003 833 (Ciba-Geigy)
EP-A-0 050 703 (Ciba-Geigy)
EP-A-0 000 053 (Merck & Co.)
EP-A-0 001 295 (Ciba-Geigy)
EP-A-0 018 072 (Beckman)
JP-A-0 019 236/1980 (Daiichi Seiyaku)
DE-A-2 747 379 (Agence Nationale...)
JP-A-0 073 729/1979 (Daiichi Pharm)
EP-A-0 004 512 (Agence Nationale...)
DE-A-2 912 865 (Yamamura)
EP-A-0 013 651 (Agence Nationale...)
EP-A-0 017 760 (Ciba-Geigy)

Life sciences, Vol. 31, pp. 1133-1140 (Bauer et al. — SMS 201-995 : A very potent and selective octapeptide analogue of somatostatin with prolonged action).

Some of these compounds are commercially available from UCB-Bioproducts, Brussels, BELGIUM

such as :

| | |
|---|---|
| B 150 | SRIF |
| B 151 | [$Tyr^1$]-SRIF |
| B 152 | Tyr-SRIF |
| B 153 | [D-$Trp^3$]-SRIF |
| B 154 | des $Asn^5$, [-D-$Trp^8$,D-$Ser^{13}$]-SRIF |
| B 155 | [D-$Trp^8$,D-$Cys^{14}$]-SRIF |
| B 156 | Cys-Phe-Phe-Trp<br>Cys-Phe-Thr-Lys |
| B 157 | Cys-Phe-Phe-D-Trp<br>Cys-Phe-Thr-Lys |
| B 158 | [$Phe^4$]-SRIF |
| B 165 | Ac-Thr-Phe-Thr-$NH_2$ |
| B 166 | Ac-Thr-Phe-Thr-Ser-$NH_2$ |
| B 167 | Ac-Lys-Thr-Phe-Thr-Ser-Ala |
| B 168 | Ac-Trp-Lys-Thr-Phe |
| B 169 | Ac-Phe-Phe-Trp-Lys-Thr-Phe |
| B 170 | Ac-Phe-Thr-Ser-Ala |
| B 171 | Ala-Lys-Asn-Phe-Phe |
| B 172 | Phe-Phe-Trp-Lys-Thr-Phe |
| B 173 | Lys-Thr-Phe-Thr-Ser-Ala |

Furthermore the products of the invention may also be substitute to GPP.
Substitutes to GPP may result from :

The combination of somatostatin or its homologues to a carrier rendering the combination immunogenic.

Somatostatin or its homologues may be is coupled to a macromolecule (carrier) through any bond created by the appropriate reaction as described in herein before. The carrier can be recognized as self or non self by the host depending upon its isology, its heterology or its transformation by any chemical modification. Typical example is the coupling of somatostatin or its homologues to any serum albumine modified or not.

Other possible carriers include hemocyanin, immunoglobulins, B2 microglobulin, Toxins (cholera, tetanos, diphteria, etc...), polysaccharides and lipopolysaccharides, natural or synthetic polyadenylic and polyuridylic acids, polyalanyl, polylysine peptides, cell membrane components (such as formaline or glutaraldehyde treated erythrocytes cell membranes). The use of any carrier is also to be made not only with somatostatin or its homologues but with any from of GPP (MDP-Somatostatin or any of their homologues).

At the level of the osidic portion of the GPP or its homologues or a glycosylated carrier, substitution can be made to create a derivative possessing any covalently bound fatty acids or lipids. These hydrophobic moities permit the insertion of any of these derivatives in suitable liposomes.

Coupling of somatostatin or its homologues to any haptenic group different from the adjuvant muramyl peptide or its homologues such as defined as hereinbefore.

Any form of coupling of somatostatin or its homologues to create polymers of various orders made by the repetition of the same moiety.

3. Furthermore the present invention covers also the combined use of any immunogenic homologue such as-described hereabove and of any immunostimulating complex.

These immunostimulating complexes are any macromolecular complex structures creating a possible vehicle to any of the immunogens described hereabove, rendering the immunogen fully accessible to the immune system of the host and interesting specifically the composition of our vaccines.

## Examples

### Example 1. Preparation of a GPP according to the invention

1. Material

The solvents and reagents were obtained from Merck (Darmstadt). The amino acids protected by the groups t-butyloxycarbonyl (t-Boc), benzyloxycarbonyl (Z) on the $\alpha$ and $\xi$-amino groups and by the benzyl group (Bzl) on the carboxyl function were obtained from UCB Bioproducts (Belgium) and Bachem (Zwitzerland). Sephadex gels were obtained from Pharmacia (Sweden). Somatostatin in its acetate form was prepared by UCB Bioproducts (Belgium). N-ethyl-N'-3-(dimethyl amino) propyl carbodiimide (EDAC) was obtained from BioRad Laboratories (USA). The trifluoroborate/methanol was from Pierce (USA) and N-acetylmuramic acid was from Janssen Pharmaceutica (Belgium). Detection of peptides was carried out

with ninhydrine by direct coloration for the amino acids having a free $NH_2$-group or after acid hydrolysis for blocked amino acids. In the second case, the detection by the chlorine-o-tolidine-KI reagent has also been used.

2. Synthesis

2.1. t-Boc-L-Ala-D-isoGln COOH 1 (Scheme 1)

1 g of t-Boc-L-Ala-D-isoGln (OBzl) was hydrogenated for 4 hours at room temperature with 2 g of Pd-PEI (Pd-Polyethyleneimine) Pierce dispersed in a solution of 50 ml tetrahydrofuran, 50 ml of distilled water and 10 ml of formic acid.

The catalyst was removed by filtration and the filtrate was evaporated under vacuum. Completion of the reaction was assessed by thin layer chromatography on silica gel plates (Merck) using ethyl Acetate, n-butanol, acetic acid, water (2 ; 1 ; 1 V/V/V) as eluant.

2.2. t-Boc-L-Ala-D-isoGln-L-Lys(N-ξ-Z) OMe 2 (Scheme 1)

150 mg (1,44 mM) of t-Boc-L-isoGln-COOH 1 was dissolved in 8 ml acetonitrile. The solution was cooled at — 20 °C and 0,2 ml (170 mg) of n-ethyl morpholine was added.

105 μl (0,8 mM) isobutyl chloroformate was added and the reaction was allowed to continue for 10 minutes.

250 mg (0,78 mM) of L-Lys(N-ξ-Z) OMe. HCl were dissolved in 3 ml of acetonitrile and added to the reaction mixture. After a reaction time of 3 hours, the solvent was evaporated under vacuum. The solid residue was redissolved in ethyl acetate-chloroform-methanol 60 ; 20 ; 20 V/V/V. After chromatography on a silica column (Lobar, Merck) eluted with the same solvent, the fractions containing the final product were evaporated under vacuum.

2.3. Trifluoroacetate of L-Ala-D-isoGln-L-Lys-(N-ξ-Z) OMe 3 (Scheme 1)

200 mg t-Boc-L-Ala-D-isoGln-L-Lys(N-ξ-Z) OMe 2 were dissolved in 10 ml of trifluoracetic acid-chloroform (3 : 1, V/V). The mixture was allowed to stand for 30 min at room temperature. The solvent was evaporated and the solid residue was redissolved in chloroform. Chloroform phase was evaporated under vacuum. 10 ml of benzene were finally added and evaporated under vacuum. The hydrolysis of the t-butyloxy-carbonyl group was followed by thin layer chromatography on silica plate with ethyl acetate-chloroform-methanol (60 ; 20 ; 20 V/V/V) as elution solvent. The purification is carried out by chromatography on a silica column with the same solvent as eluent.

2.4. N-Acetyl-(1-Bzl-4,6-0-Bzi)muramyl-L-Ala-D-isoGln-L-Lys(N-ξ-Z) OMe 4 (Scheme 3)

The partially protected peptide 3 was coupled to a protected N-acetyl muramic acid such as N-acetyl-1-benzyl-4,6-0-benzylidene muramic acid C in a stoechiometric quantity, according to the method of Ozawa Jeanloz (1965) and Lefrancier et al. (1978) with the modifications described under section 2.4 a, b, c, f and g.

a) N-acetyl-1-benzyl-D-pyrannoglucosamine A (Scheme 2)

(Modified method of Ozawa and Jeanloz (1965)).

47 g (0,2 mole) of N-acetyl-D-pyrannoglucosamine were added to a solution of chlorhydric acid in benzyl alcohol (2 % V/V). The mixture was stirred for 2 hours at 70-80 °C. The solution was then allowed to stand for 30 minutes at room temperature. Two liters of ethylether were then added and the mixture was stirred for one more hour. The mixture was then filtred and the precipitate was washed with petroleum ether 60-80 °C. The filtrate was similarly treated with another liter of ethyl ether. The solid residues were pooled and recristallized in ethanol. White cristals 50-55 g (91-98 % yield) with a melting point at 180-6 °C were obtained. The elementary analysis and the NMR spectrum are correct (see table 1).

b) N-acetyl-1-benzyl-4,6-0-benzylidene-D-pyranno glucosamine B (Scheme 2)

10 g (0,07 mole) of powdered $ZnCl_2$ was added to a mixture of 13 g (0,04 mole) of A and 200 ml benzaldehyde in 150 ml of ethylether. The solution was stirred during 20 hours at room temperature after which a solution of 3 g of ammonium chloride was added to the medium. The mixture was then diluted with distilled water cooled at 0 °C. The precipitate was filtered and washed twice with a solution of 2-propanol in water (10 % V/V). 12 g (78 % yield) of white cristal with a melting point at 224-250 °C were obtained. The elementary analysis is correct. The NMR-spectrum data are given in table 1.

c) N-acetyl-1-benzyl-4,6-0-benzylidene muramic acid C (Scheme 2)

3,2 g (0,01 mole) of B dissolved in 200 ml of dioxane was added gradually under stirring to 1,15 g (0,04 mole) of natrium hydride 80 % in oil. The mixture was stirred for one hour at 90-100 °C. The temperature is reduced to 70 °C and 4,5 g (0,04 mole) of 2-chloropropionic acid were added. The reaction was allowed to continue for 20 hours at 65-75 °C with magnetic stirring. The mixture was then cooled to 0 °C and 200 ml of distilled water were carefully added dropwise. The dioxane was evaporated under reduced pressure and the aqueous solution was added to a mixture of concentrated chlorohydric acid in ground ice (pH 1). The precipitate was extracted with chloroform. The organic phase was washed with water, dried on magnesium sulfate and finally evaporated under vacuum. The oily residue was cristallized with petroleum ether 60-80 °C.

The solid residue was then redissolved in 50 ml of chloroform. The insoluble fraction was filtred and amounted to 1 g (21 % yield) of N-acetyl-1-benzyl-4,6-0-benzylidene muramic acid (224-8 °C). The chloroform was evaporated and the solid phase was recristallized in a mixture of toluene dioxane. 0,8 g of precipitate (17 % yield) was obtained wich corresponded to N-acetyl-1-benzyl-4,6-0-benzylidene muramic acid (M.P. : 274-6 °C) (M.P. 243-4°, Ozawa and Jeanloz, 1965). The soluble fraction was dried, separated from the solvant by evaporation and the residue washed with toluene. 1,3 g (28 % yield) of a mixture of α and β forms of the protected sugar was finally obtained. Elementary analysis was correct. NMR spectrum data are given in table 1.

An alternative procedure for obtaining fully protected N-acetyl muramic acid was the following :

d) Benzyl (N-acetyl-1-benzyl)-muramate D (Scheme 2)

2 g (6,8 mmoles) of N-acetyl muramic acid were dissolved in 100 ml of hydrochloride in benzylic alcohol (2 % W/W). The mixture was heated for 3 hours at 90-100 °C. The solvent was evaporated under vacuum and the residue was washed with ethylether. The final product (3 g-93 % yield) had a melting point of 138-144 °C. It was found to be sufficiently pure for the following operation. Elementary analysis was correct : v CO = 1 740 $cm^{-1}$ (ester). NMR spectrum data are given in table 1.

e) Benzyl-(N-acetyl-1-benzyl-4,6-0-benzylidene)-muramate E (Scheme 2)

3 g (6,34 mmoles of D, and 3 g (20 mmoles) of $ZnCl_2$ are dissolved in 30 ml of benzaldehyde and 30 ml ethylether and were treated in accordance with the process previously described (section 2.4 b).

The purification of the products was performed as in section 2.4. b. The solid residue was washed with chloroform. The yield of the operation was found to be 84 % yield, i. e. 3 g of the purified product (M.P. : 222-234 °C). The elementary analysis was correct.

f) N-acetyl-1-benzyl-4,6-0-benzylidene muramic acid F (Scheme 2)

3 g (5,3 mmoles) of E were added to 150 ml of sodium hydroxide 0.5 M and 150 ml of dioxane. The mixture was stirred for 24 hours at room temperature. Finally, the mixture was heated under reflux for 15 minutes.

The solution was acidified to pH 1. Purified α and β forms of the protected sugar have been purified such as the operative mode described in section C.

The following yield were respectively obtained :

250 mg of the α form (10 % yield)
750 mg of the β form (30 % yield)
1 400 mg of the α + β mixture (56 % yield)

g) (N-acetyl-1-benzyl-4,6-0-benzylidene) Mur-L-Ala-D-isoGln-L-Lys(N-ξ-Z) OMe 4 (Scheme 3)

1 g (2,6 mM) of C was dissolved in 15 ml dimethylformamide (DMF), precooled to — 20 °C. 0,25 ml (2,25 mM) of N-methylmorpholine and 0,3 ml (2,25 mM) isobutyl chloroformate then a solution of L-Ala-D-isoGln-L-Lys(N-ξ-z) OMe. TFA 3 (2,25 mM) in 15 ml of DMF were successively added. The reaction was performed with stirring for 4 hours at — 20 °C. Finally 2,25 ml of $KHCO_3$ were added and the product was precipitated, washed with water and freeze dried. Elementary analysis was correct.

2.5. N-Acetyl-Mur-L-Ala-D-isoGln-L-Lys(N-ξ-Z) OMe MTP 5 (Scheme 3)

The protected peptide 4 was then treated by acetic acid and then hydrogenated essentially in the same conditions described under section 1.

Purification of the peptide was performed by chromatography on a silica column with a pyridine-n-butanol-acetic acid-water mixture (10 : 15 : 3 : 12 ; V/V/V/V) as eluting solvent. Elementary analysis was correct.

2.6. O-methylated somatostatin (SRIF-OME) G

50 mg of purified somatostatin (SRIF) were dissolved in 2 ml of trifluoroborate in methanol (14 % W/V) and allowed to stand at room temperature for 16 hours. Completion of the reaction was followed by thin layer chromatography with a n-butanol-pyridine-acetic-acid-water mixture (4/1/1/1 ; V/V/V/V) as eluting solvent. 10 ml of ethylether dried on sodium were added at the end of the reaction and the solvent was evaporated under vacuum. The solid product was redissolved in 2 ml of acetic acid. In water (30 % V/V) and submitted to a filtration on Sephadex G 10 in the same solvent. The fractions containing the methylated somatostatin were pooled and freeze dried (30 mg, 60 % yield). The powder was then redissolved in distilled water and relyophilized. Chemical integrity of tryptophan residue in the methylated somatostatin was controled by ultraviolet spectroscopy. Elementary analysis was correct.

2.7. Coupling of Muramyl dipeptide (MDP) with methylated somatostatin (SFIF-OMe), F (Scheme 3)

a) 25 mg (0,02 mmole) of the chlorhydrate form of the methylated somatostatine G dissolved in 1,5 ml of distilated water were coupled for 2 hours with 8,9 mg of MDP (0,018 mmole), by means of 60 mg (0,4 mmole) of EDAC (N-ethyl-N'-(3-dimethyl-amino-propyl) carbodiimide, the pH being maintained between 4,5 and 5,0 by addition of diluted chlorhydric acid. After that, the reaction was allowed to continue at room temperature for 16 hours. Then a few drops of glacial acetic acid were added to the solution and the precipitate obtained centrifuged. The supernatant was recovered and freeze dried. The powder was redissolved in 1 ml of the mixture of n-butanol-pyridine-acetic-acid water 4/1/1/2 ; V/V/V/V and purified on a SI 60 Lobar Merck column with the same mixture as eluting solvent.

b) 25 mg (0,02 mmole) of SRIF-OMe and 8,9 mg of MDP (0,018 mmole), 60 mg (0,4 mole) of EDAC and 2,2 mg (0,018 mmole) of N-hydroxysuccinimide were dissolved in 1 ml of 0,02 M phosphate buffer pH 7,8.

The mixture was allowed to react for 16 hours at room temperature. Purification of the peptide was performed in the conditions similar to these of section (a).

2.8. Coupling of a muramyl tripeptide analog with somatostatine (SRIF) F' (Scheme 3)

60 mg of EDAC were dissolved in 50 µl of distilled water and added to a solution of 25 mg of somatostatine (chlorhydrate form) in 1 ml of distilled water. The pH was maintained between 4.5 and 5.0 mg of MTP (N-acetyl muramyl-L-Ala-D-isoglutamyl-L-lysine OMe) were added and the reaction was allowed at room temperature for 2 hours.

The purification of the coupled product was performed as in section (a).

Example 2

Treatment of young bulls with the growth promoting peptides (GPP).

After a 2 months observation period (ajustment to husbandry conditions and base line measurements), thirty young bulls of around 250 kg were observed for 7 months.

They were injected four times with 100 µg of GPP or a mixture of equivalent amounts of muramyl di- or triptide and SRIF. Samples were solubilized in physiological medium supplemented with 40 % W/V polyethyleneglycol 6000. Intramuscular injections in the neck of animals were performed with 3 ml of the solution at days 0, 10, 30 and 60 of the 7 months period. During the seventh months treatment period the animals received a concentrate ad libitum. Each month, the animals were weighed and blood samples were taken for further analyses. The results concerning the parameters studied in this zootechnical experiment were summarized in the following.

1) Statistical analysis of the life weight gain of control and treated animals

Variance analysis of the body live weight gain has been performed on 27 animals alive on September 27, 1984 (16 controls and 11 treated animals). At that time, the difference of life weight gain between the 16 control animals and the 11 treated animals was 31.3 kg per animal, i. e. 11 % over controls.

This difference is statistically significant at P 0.10.

If the performance of the 3 animals accidentally dead during the experiment were being taken into additional consideration, the difference in the life weight gain between control and treated animals would be found to be 34.9 kg per animal, in favour of the treated ones.

The deviation measured between the life gain of treated animals of batch 5 (SRIF, MTP treated) and 3 (SRIF, MDP treated) is low (5.7 kg) and without any statistical significance.

During the experimental period, the food intake of the treated animals was 50 kg higher than that of the control one.

For a same food intake, the total weight gain of a treated animal is 30 kg higher than that of control

animals.

The food intake per kg live weight gain decrease from 5.46 (controls) to 5.06 kg (treated ones) (representing 400 g less in favor of the treated animals).

Analysis of the graphs shows that the treatment gives moderate results during the first two months, a steeper increase in weight gain in the following 3 months and an apparent slow down in the remaining period.

Carcass analysis (on rib segment and carcass : relative composition in muscles, adipo-connective tissues and bones) was performed. The treated animals exhibited similar proportions in these compartments as compared to the controls.

Treatment of animals by GPP.

Survey of biological parameters :

The following parameters have been studied throughout to experimental :
— blood glucose
— T4 (RIA)
— T3 (RIA)
non difference between treated and control animals.
— Testosterone (RIA)

Growth hormone (RIA) has been measured at the third month after the initial GPP injection.

No statistical difference between treated and control animals.

Anti-SRIF antibodies

The detection of free binding sites for SRIF has been performed at various period after initial treatment using $^{125}$I-Tyr-SRIF as tracer. A charcoal separation was used for measurement of bound and free radioactivity after a 48 h incubation period, at 4 °C of the tracers with various dilutions of the serum samples.

Specific binding is noted 3 months after the initial GPP injection, and is mainly restricted to the animals treated by the GPP(s).

Indeed ninty percents of animals treated with GPP-exhibited binding sites for $^{125}$I-Tyr-SRIF Titers never exceed 1/200 (final dilutions) for GPP treated animals and remain stable during all the experimental period.

(See Table I and schemes, p. 12-15)

Table 1

NMR spectra
Chemical displacements (ppm)
(Internal reference : HMDS — solvent : DMSO-$d_6$)

General formula 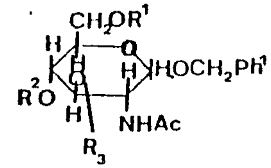

| $R^1$ | $R^2$ | $R^3$ | Isomer | NH | $Ph^2$ or $Ph^3$ | $Ph^1$ | -CH-Ph | H-1 | -CH-$CH_3$ | H-2 | H-3 | H-4 | H-5 | $nCH_2$ (n=1,2) | $CH_3$-CONH- | $CH_3$-CH- |
|---|---|---|---|---|---|---|---|---|---|---|---|---|---|---|---|---|
| H | H | H | | | | | | | | | | | | | | |
| $Ph^2CH$ | $Ph^2CH$ | H | α<br>β | 7.70<br>8.02 | 7.21 | 7.18 | 5.41 | 4.95 | 4.43 | 4.62 | 4.03 | 3.31 | 3.80 | 3.12 | 1.71 | - |
| $Ph^2CH$ | $Ph^2CH$ | -CH($CH_3$)-COOH | α<br>β | 7.72<br>7.98 | 7.14 | 7.12 | 5.50<br>5.37 | 4.84<br>4.68 | 4.45 | | 3.64 - 4.15 | | | 3.56 | 1.71 | 1.10<br>1.05 |
| H | H | -CH($CH_3$)-$COOCH_2Ph^3$ | α<br>β | 7.42<br>7.59 | 7.13 | 7.03 | - | 5.00<br>4.90 | 4.21 | | 3.26 - 4.74 | | | 3.16 | 1.66<br>1.59 | 1.20<br>1.13 |
| H | H | -CH($CH_3$)-COOH | β | 7.66 | - | - | - | 5.34 | 4.36 | | 3.08 - 3.50 | | | 3.05 | 1.74 | 1.15 |

Scheme 1

t-Boc-L-Ala-D-isoGln(OBzl)

Pd -PEI

t-Boc-L-Ala-D-isoGln COOH

1

1) ClCOOisoBu, N-ethylmorpholine

2) L-Lys (N-ε-Z)OMe

t-Boc-L-Ala-D-isoGln-L-Lys (N-ε-Z)OMe

2

$CF_3COOH$ in $CHCl_3$

TFA.$H_2$N-L-Ala-D-isoGln-L-Lys (N-ε-Z)OMe

3

Scheme 2

1.

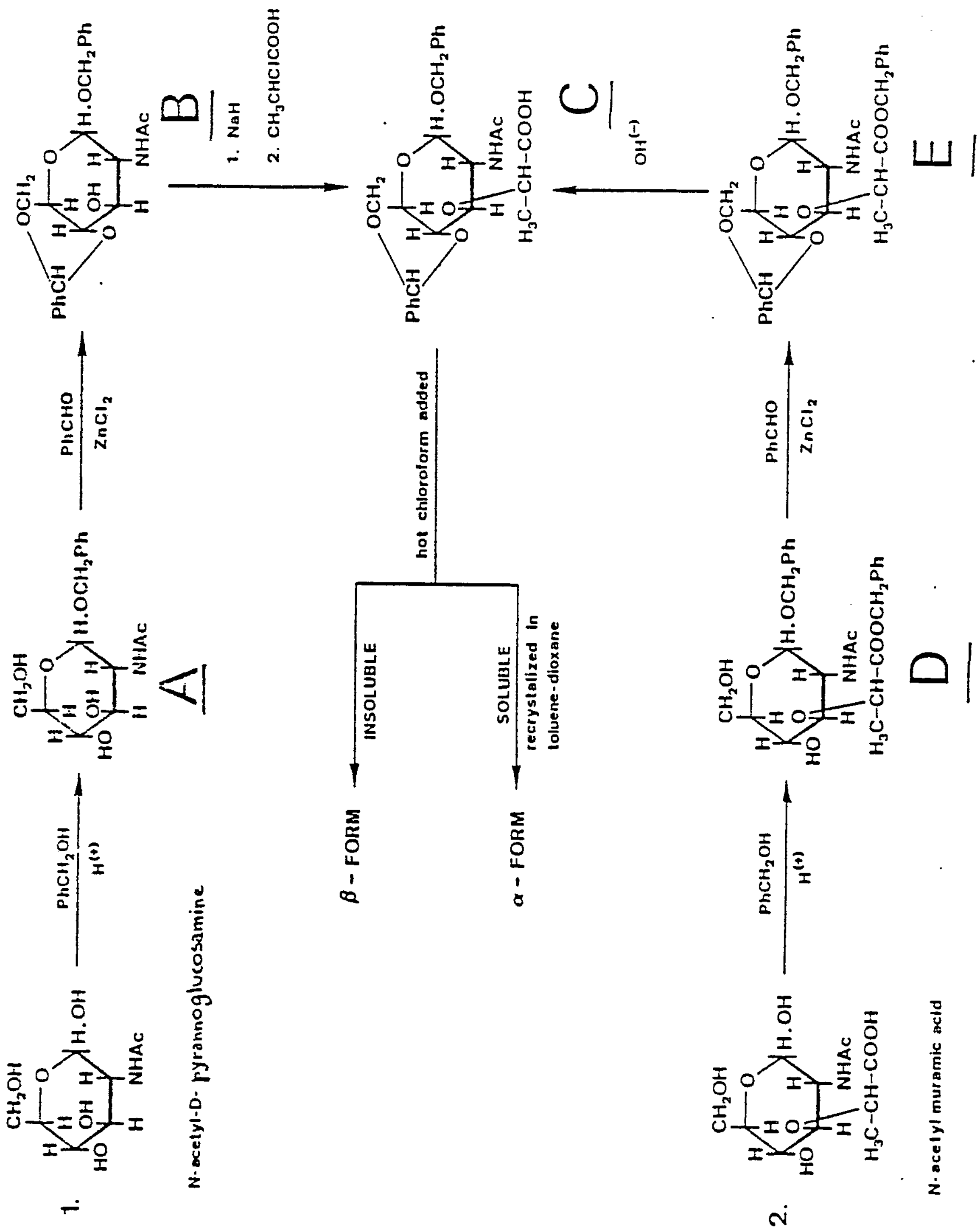


N-acetyl-D-pyranoglucosamine

A

B

C

2.

N-acetyl muramic acid

D

E

Scheme 3

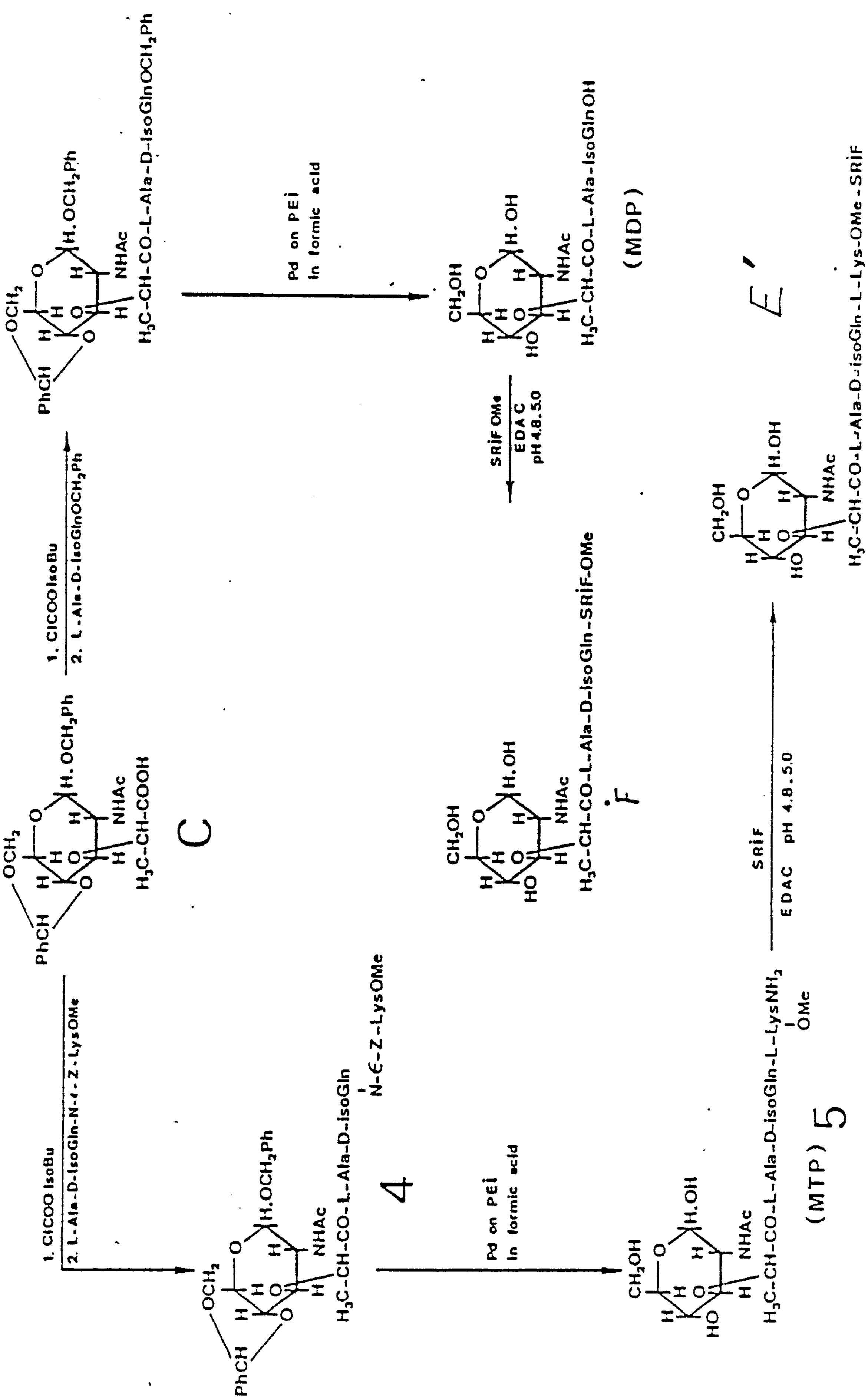


C

(MDP)

F

E'

4

(MTP) 5

**Claims**

1. Compound for the promotion of growth and/or milk production of domestic animals, characterized in that it consists in somatostatin or a homologue thereof, in the natural or oxidized state or in the pro-form or in the pre-proform coupled, possibly over a bridging element, with at least one muramyl-peptide.

2. Compound according to claim 1 characterized in that it consists in N-acetylmuramyl-L-alanyl-D-isoglutamine or a homologue thereof coupled with somatostatin or a homologue in its natural or oxidized state or in the pro-forme or its pre-proform.

3. Compound according to claim 1 characterized in that it consists in an ester, such as the $C_1$-$C_{10}$alkyl-ester of N-acetylmuramyl-L-alanyl-D-isoglutaminyl-L-lysine coupled at the carboxylic group to somatostatin or to an ester of somatostatin, such as the $C_1$-$C_{10}$alkyl-ester thereof, in the natural or oxidized state or in the pro-form or its pre-proform.

4. Compound according to claim 1 characterized in that a muramyl-dipeptide is coupled at its carboxylic end to the carboxylic group of somatostatin or of an ester of somatostatin, in the natural or oxidized state or in the pro-form or its pre-proform.

5. Compound according to claim 1 characterized in that it corresponds to the formula

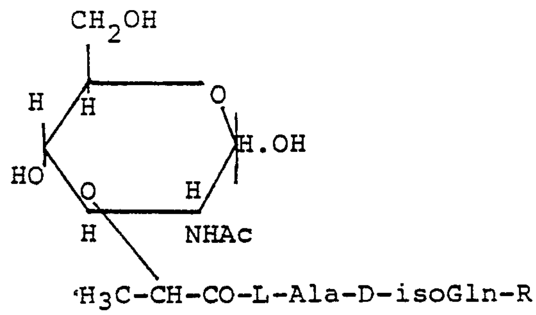

wherein R represents the group SRIF-OMe or the group

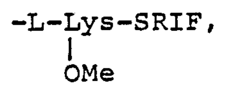

SRIF representing somostatin or a homologue thereof.

6. Compound according to claim 1 characterized in that the homologues of N-acetylmuramyl-L-alanyl-D-isoglutamine are selected from the group comprising :

substitution products of hydrogen of 1-hydroxy group or 1-hydroxy of the saccharide ring by alkyl, aryl, alkylaryl, arylalkyl or by amino group wherein said substitution products have functional groups such as hydroxy, amine, carboxamide, sulfonamide, ether, thioether ;

substitution products of hydrogen of 6-hydroxy function or 6-hydroxy of the saccharide ring possibly protected by carboxamide, sulfonamide, ether, thioether, ester, thioester or replaced by amine, possibly bearing aryl, phenyl, heterocyclic, alkyl, carbonyl, amino, sulfhydryl, peroxyl groups ;

compounds wherein the alanyl residue of the muramyl peptide is substituted by arginyl, asparagyl, aspartyl, glutamyl, glycyl, histidyl, hydroxyprolyl, isoleucyl, leucyl, lysyl, methionyl, phenylalanyl, prolyl, seryl, threonyl, tryptophanyl, tyrosyl, valyl, aminobutyryl residues ;

compounds wherein the carboxyl function of the D-isoglutaminyl residue is esterified or substituted by any other amino acid having an other carboxylic function, or a lysyl residue of a muyramyl tripeptide is substituted by an amino acid having supplementary to their amino and carboxylic functions, an amino, hydroxyl or acid function, such amino acid possibly being acid, glutamic acid, ornithine, tyrosine.

7. Compound according to claim 1 characterized in that the homologues of somatostatin are selected from the group comprising :

substitution products wherein at least one is substitued in any position of the chain by a natural or non natural L- or D-aminoacid such as taurine or aminobutyric acid ;

DES-forms as well as cyclic forms (non reductible cyclic analogs) and pro-forms, such as S.S. 28 amino acids form and SS DES-25 proform under their reduced or oxydized forms ;

other forms with additional $NH_2$-terminal aminoacid resulting from peptide synthesis or DNA recombinant techniques either as a molecule presenting a portion of the natural presequence or as molecules exhibition any sort of substitution in any position of this proform ;

molecules resulting from the protection of reactive groups on the side chains of the aminoacids :

glycosylated somatostatin analogs resulting from the glycosylation of 5-asparagine, 13-serine, 10- and 12-threonine or any other position provided that this position is occupied by one of these 3 amino acids ;

substitution products of sulfur in cysteine residue by selenium ;

derivatisation products of the somatostatin portion of any GPP resulting from the use of polymer of

various order of somatostatin itself retaining at least one and preferably multiple immunogenic determinants of somatostatin.

8. Process for the preparation of a compound according to any one of the preceding claims, characterized in that muramyl-peptide or a homologue thereof is condensed with somatostatin or a homologue in the natural or oxidized state or in the proform or its preproform, in the presence of an appropriate condensation reagent.

9. Process according to claim 8 characterized in that N-acetylmuramyl-L-alanyl-D-isoglutamine or a homologue thereof is reacted with somatostatin or a homologue thereof in a ratio between 1/1 and 3/1.

10. Preparation for the promotion of growth and/or milk production of domestic animals characterized in that it contains at least one compound according to anyone of claims 1 to 7.

11. Preparation according to claim 10 characterized in that it contains the active compound in an effective quantity of from 10 to 500 µg per dose, depending on the species of recipient and the type of immunization procedure chosen.

12. Preparation according to claim 10 characterized in that it includes one or more excipients and support substances rendering the preparation adequate for parenteral injection such as intramuscular or subcutaneous injection or bootsing injections or for oral or nasal spray administration.

13. Use of the compound according to anyone of the claims 1 to 7 for the promotion of growth and/or milk production of domestic animals, through an immunogenic action on the somatostatin secretion which implies an increased secretion of the endogenic growth hormone.

**Patentansprüche**

1. Verbindung zur Förderung des Wachstums und/oder der Milchproduktion von Haustieren, dadurch gekennzeichnet, dass sie aus Somatostatin oder einem seiner Homologen, in natürlicher oder oxydierter Form oder in der pro-Form oder in der pre-pro-Form, besteht, das mit mindestens einem Muramylpeptid, gegebenenfalls über ein Brückenelement, verbunden ist.

2. Verbindung nach Anspruch 1, dadurch gekennzeichnet, dass sie aus N-Acetylmuramyl-L-Alanyl-D-Isoglutamin oder einem seiner Homologen besteht, dass mit Somatostatin oder einem Homologen, in natürlicher oder oxydierter Form oder in der pro-Form oder in der pre-pro-Form, verbunden ist.

3. Verbindung nach Anspruch 1, dadurch gekennzeichnet, dass sie aus einem Ester, wie $C_1$-$C_{10}$ Alkylester, des N-Acetylmuramyl-L-Alanyl-D-Isoglutaminyl-L-Lysin besteht, das über den Carboxylrest mit Somatostatin oder einem Ester von Somatostatin, wie ein $C_1$-$C_{10}$ Alkylester von Somatostatin, in natürlicher oder oxydierter Form oder in der pro-Form oder dessen pre-pro-Form verbunden ist.

4. Verbindung nach Anspruch 1, dadurch gekennzeichnet, dass ein Muramyl-dipeptid über seinen Carboxylrest mit dem Carboxylrest von Somatostatin oder einem Ester von Somatostatin, in natürlicher oder oxydierter Form oder in der pro-Form oder dessen pre-pro-Form, verbunden ist.

5. Verbindung nach Anspruch 1, dadurch gekennzeichnet, dass sie der Formel

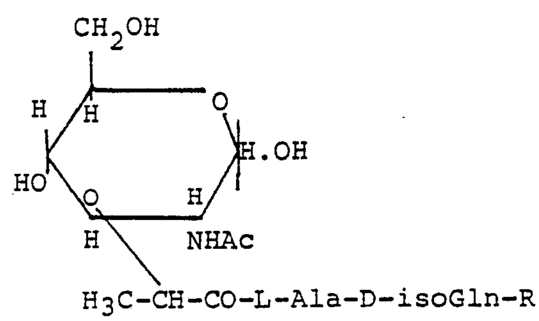

entspricht, in der R die Gruppe SRIF-OMe, oder die Gruppe

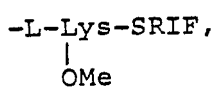

darstellt, wobei SRIF für Somatostatin oder einen seiner Homologen steht.

6. Verbindung nach Anspruch 1, dadurch gekennzeichnet, dass die Homologen von N-Acetylmuramyl-L-Alanyl-D-Isoglutamin aus der Gruppe ausgesucht sind, die enthält :

Produkte aus der Substitution des Wasserstoffs der 1-Hydroxygruppe oder 1-Hydroxygruppe des Saccharidrings durch eine Alkyl-, Aryl-, Alkylaryl-, Arylalkyl- oder Aminogruppe, wobei die besagten Substitutionsprodukte funktionnelle Gruppen aufweisen, wie die Hydroxy-, die Amin-, die Carboxamid-, die Sulfonamid, eine Äther- oder Thioäthergruppe ;

Produkte aus der Substitution des Wasserstoffs der 6-Hydroxygruppe oder 6-Hydroxygruppe des

Saccharidrings, gegebenenfalls durch Caboxamid-, Sulfonamid-, Äther-, Thioäther-, Ester-, Thioestergruppe geschützt, oder durch eine Amingruppe, die gegebenenfalls eine Aryl-, Phenylgruppe, heterocyclische Gruppe, Alkyl-, Carbonyl-, Amino-, Sulfhydryl-, Peroxylgruppe ersetzt ;

Verbindungen, in denen der Alanylrest des Muramylpeptid durch einen Arginyl-, Asparagyl-, Aspartyl-, Glutamyl-, Glycyl-, Histidyl-, Hydroxyprolyl-, Isoleucyl-, Leucyl-, Lysyl-, Methionyl-, Phenylalanyl-, Prolyl-, Seryl-, Threonyl-, Tryptophanyl-, Tyrosyl-, Valyl-, Aminobutyrylrest substituiert ist ;

Verbindungen, in denen die Carboxylgruppe des D-Isoglutaminylrests durch irgend eine andere eine Carboxylgruppe tragende Aminosäure verestert oder substituiert ist, oder in denen ein Lysylrest eines Muramyltripeptids durch eine Aminosäure substituiert ist, die zusätzlich zu ihrer Amino- oder Carboxylgruppe eine Aminogruppe, Hydroxylgruppe oder Säurefunktion trägt, wobei die Aminosäure gegebenenfalls aus (Aspartin) säure, Glutaminsäure, Ornithin, Tyrosin besteht.

7. Verbindung nach Anspruch 1, dadurch gekennzeichnet, dass die Homologen von Somatostatin aus der Gruppe ausgesucht sind, die enthält :

Substitutionsverbindungen, in denen mindestens ein in irgend einer Position der Kette durch eine natürliche oder nicht natürliche L- oder D-Aminosäure, wie Taurin oder Aminobuttersäure, substituiert ist ;

DES-Formen sowie auch zyklische Formen (nicht reduzierbare zyklische Analogen) und pro-Formen, wie SS.28 Aminosäure und SS.DES-25 pro-Form in der reduzierten oder oxydierten Form ;

andere Formen mit zusätzlicher endständiger $NH_2$-Gruppe tragender Aminosäure, die aus der Peptidsynthese oder DNS-Rekombinationstechnik entstehen, entweder als ein Teil der natürlichen Sequenz aufweisendes Molekül oder als Moleküle, die jede Art der Substitution in jeder Position dieser pro-Form aufweisen ;

Moleküle die Schutzgruppen auf den reaktiven Gruppen der Seitenketten der Aminosäuren tragen ;

Glycosylierte Somatostatinanaloge, die aus der Glycosylierung von 5-Asparagin, 13-Serin, 10-Threonin und 12-Threonin entstehen, oder aus der Glycosylierung in jeglicher anderer Position, unter der Bedingung, dass diese Position durch eine dieser 3 Aminosäuren besetzt ist ;

Verbindungen aus der Substitution des schwefels im Cysteinrest durch Selen ;

Derivate des Somatostatinteils, eines jeden GPP, die aus der Verwendung eines Somatostatin-Polymers von unterschiedlichem Grad entstehen, die mindestens eine und vorzugsweise mehrere immunogene Determinanten des Somatostatins tragen.

8. Verfahren zur Herstellung einer Verbindung nach einem der vorherigen Ansprüche, dadurch gekennzeichnet, dass ein Muramylpeptid oder ein seiner Homologen mit Somatostatin oder einem Homologen in der natürlichen oder oxydierten Form oder in der pro-Form oder dessen pre-pro-Form, in Gegenwart eines geeigneten Kondensationsmittels, kondensiert wird.

9. Verfahren nach Anspruch 8, dadurch gekennzeichnet dass N-Acetylmuramyl-L-Alanyl-D-Isoglutamin oder ein seiner Homologen mit Somatostatin oder einem seiner Homologen, im Verhältnis von 1/1 bis 3/1 umgesetzt wird.

10. Präparation zur Förderung des Wachstums und/oder der Milchproduktion von Haustieren dadurch gekennzeichnet, dass sie mindestens eine Verbindung nach einem der Ansprüche 1 bis 7 enthält.

11. Präparation nach Anspruch 10 dadurch gekennzeichnet, dass sie die aktive Verbindung in einer effectiven Menge von 10 bis 500 µg pro Dosis enthält, abhängig von der Art des Empfängers und der Art des gewählten Immunisationsprozesses.

12. Präparation nach Anspruch 10 dadurch gekennzeichnet, dass sie ein oder mehrere Ekzipienten und Trägersubstanzen enthält, die die Präparation zur parenteralen Injection, wie intramuskuläre, subkutane Injektion oder Schröpfen, oder zur mündlichen Verabreichung oder als Nasenspray geeignet machen.

13. Verwendung der Verbindung nach einem der Ansprüche 1 bis 7 zur Förderung des Wachstums und/oder der Milchproduktion von Haustieren durch einen immunogenen Einfluss auf die Somatostatinsekretion des endogenen Wachstumshormons, wodurch eine Erhöhung der Sekretion des endogenen Wachsturmshormons erreicht wird.

**Revendications**

1. Composé destiné à promouvoir la croissance et/ou la production de lait des animaux domestiques, caractérisé en ce qu'il est constitué par la somatostatine ou un homologue de celle-ci, à l'état naturel ou oxydé ou sous la pro-forme ou sous la pré-pro-forme, couplée à au moins un muramyl-peptide, éventuellement via un élément de pont.

2. Composé selon la revendication 1 caractérisé en ce qu'il consiste en N-acétylmuramyl-L-alanyl-D-isoglutamine ou en un homologue de celle-ci, couplée à la somatostatine ou à un homologue, à l'état naturel ou oxydé ou sous la pro-forme ou la pré-pro-forme.

3. Composé selon la revendication 1 caractérisé en ce qu'il consiste en un ester, tel que l'ester $C_1$-$C_{10}$ alkylique de N-acétylmuramyl-L-alanyl-D-isoglutaminyl-L-lysine couplée par le groupe carboxylique à de la somatostatine ou à un ester de somatostatine, tel que l'ester $C_1$-$C_{10}$ alkylique de celle-ci, à l'état naturel ou oxydé ou sous la pro-forme ou sa pré-pro-forme.

4. Composé selon la revendication 1 caractérisé en ce qu'un muramyl-dipeptide est couplé par son extrémité carboxylique au groupe carboxylique de la somatostatine ou d'un ester de somatostatine, à l'état naturel ou oxydé ou sous la pro-forme ou sa pré-pro-forme.

5. Composé selon la revendication 1 caractérisé en ce qu'il correspond à la formule

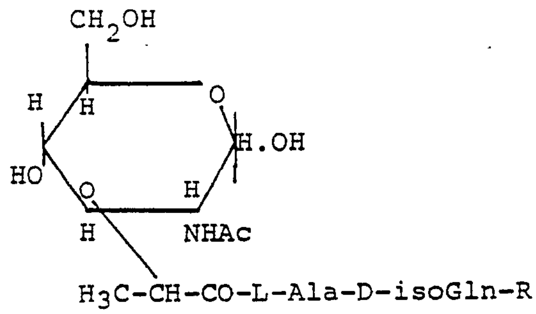

dans laquelle R représente le groupe SRIF-OMe ou le groupe

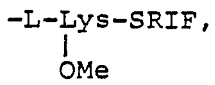

SRIF représentant la somatostatine ou un homologue de celle-ci.

6. Composé selon la revendication 1 caractérisé en ce que les homologues de la N-acétylmuramyl-L-alanyl-D-isoglutamine sont choisis dans le groupe comportant :

les produits de substitution de l'hydrogène du groupe 1-hydroxy ou 1-hydroxy du cycle de la saccharide par un groupe alkyle, aryle, alkylaryle, arylalkyle ou par un groupe amino, les produits de substitution ayant des groupes fonctionnels tels que hydroxy, amine, carboxamide, sulfonamide, éther, thioéther ;

les produits de substitution de l'hydrogène de la fonction 6-hydroxy ou 6-hydroxy du cycle de la saccharide, éventuellement protégés par un groupe carboxamide, sulfonamide, éther, thioéther, ester, thioester ou remplacés par une amine portant éventuellement des groupes aryle, phényle, hétérocyclique, alkyle, carbonyle, amino, sulfhydryle, peroxyle ;

des composés dans lesquels le reste alanyle du muramylpeptide est substitué par des restes arginyle, asparagyle, aspartyle, glutamyle, glycyle, histidyle, hydroxyprolyle, isoleucyle, leucyle, lysyle, méthionyle, phénylalanyle, propyle, séryle, thréonyle, tryptophanyle, tyrosyle, valyle, aminobutyryle ;

des composés dans lesquels la fonction carboxylique du reste D-isoglutaminyle est estérifiée ou substituée par tout autre acide aminé comportant une autre fonction carboxylique, ou dans lequels un reste lysyle d'un muramyl-tripeptide est substitué par un acide aminé comportant, en plus de (ses) fonctions amino et carboxylique, une fonction amino, hydroxyle ou acide, un tel acide aminé étant éventuellement l'acide (aspartique), l'acide glutamique, l'ornithine, la tyrosine.

7. Composé selon la revendication 1 caractérisé en ce que les homologues de somatostatine sont choisis dans le groupe comportant :

les produits de substitution dans lesquels un au moins est substitué dans une position quelconque de la chaîne par un acide aminé lévogyre ou dextrogyre naturel ou non, tel que la taurine ou l'acide aminobutyrique ;

les formes DES ainsi que les formes cycliques (analogues cycliques non réductibles), et les pro-formes, telles que la forme SS.28 aminoacide et la pro-forme SS.DES.25, à l'état réduit ou oxydé ;

d'autres formes comportant d'autres acides aminés à groupement $NH_2$ terminal, résultant de la synthèse des peptides ou des techniques de ADN recombinant, en tant que molécules présentant une partie de la préséquence naturelle ou en tant que molécules présentant tout type de substitution en toute position de cette pro-forme ;

les molécules résultant de la protection de groupes réactifs sur les chaînes latérales des acides aminés ;

des analogues de somatostatine glycosylée résultant de la glycosylation de 5-asparagine, 13-sérine, 10-thréonine et 12-thréonine ou en toute autre position, pourvu que cette position soit occupée par l'un de ces 3 acides aminés ;

les produits de substitution du soufre par le sélénium, dans le reste cystéine ;

les produits dérivés de la partie somatostatine de tout GPP résultant de l'utilisation d'un polymère de divers ordres de la somatostatine elle-même portant au moins un et, de préférence, de multiples déterminants immunogéniques de la somatostatine.

8. Procédé pour la préparation d'un composé selon l'une quelconque des revendications précédentes caractérisé en ce qu'on condense du muramyl-peptide ou un homologue de celui-ci avec de la somatostatine ou un homologue, à l'état naturel ou oxydé ou sous la pro-forme ou sa pré-pro-forme, en présence d'un agent de condensation adéquat.

9. Procédé selon la revendication 8 caractérisé en ce qu'on fait réagir de la N-acétylmuramyl-L-alanyl-D-isoglutamine ou un homologue de celle-ci avec de la somatostatine ou un homologue de celle-ci dans le rapport compris entre 1/1 et 3/1.

10. Préparation destinée à promouvoir la croissance et/ou la production de lait des animaux domestiques caractérisée en ce qu'elle contient au moins un composé selon l'une quelconque des revendications 1 à 7.

11. Préparation selon la revendication 10 caractérisée en ce qu'elle contient le composé actif en une quantité efficace allant de 10 à 500 µg par dose, en fonction du type de receveur et du type de procédé d'immunisation choisi.

12. Préparation selon la revendication 10 caractérisée en ce qu'elle contient un ou plusieurs excipients et substances de support rendant la préparation adéquate pour l'injection parentérale, telle que intra-musculaire ou sous-cutanée ou la scarification ou pour l'administration orale ou par spray nasal.

13. Utilisation du composé selon l'une quelconque des revendications 1 à 7 destiné à promouvoir la croissance et/ou la production de lait des animaux domestiques, grâce à une action immunogénique sur la sécrétion de somatostatine, ce qui implique un accroissement de la sécrétion de l'hormone de croissance endogène.